# EUROPEAN PATENT APPLICATION

(11) **EP 4 546 182 A1**
(43) Date of publication of application: **30.04.2025**
(21) Application number: 23205904.8
(22) Date of filing: 25.10.2023
(51) Int. Cl.: G06F 21/32, G06F 1/16, G06F 3/01, G06F 21/35, H04L 9/40, H04W 12/06, H04W 12/68, H04W 12/08, A61B 5/00

(54) **ACCESS CONTROL METHOD BEING PERFORMED BY A WEARABLE DEVICE**

(71) Applicant: dormakaba Schweiz AG, 8623 Wetzikon (CH)
(72) Inventor: MUELLER, Markus, 8153 Rümlang (CH)
(74) Representative: Balder IP Law, S.L.

(57) **Abstract**

In accordance with a first aspect of the present invention, there is proposed a computer-implemented access control method being performed by a wearable device, the wearable device being secured to a user's body part, with particular emphasis on a user's arm and/or hand. The method incorporates obtaining sensor data from a biological sensor of the wearable device, either in a continuous or repetitive manner, wherein the sensor data collected provides information about the characteristics of the user's blood circulating in the body part or, in some instances, where the blood characteristics are determined based on the sensor data. The method includes determining a change in the characteristics of the user's blood where the magnitude of the change exceeds a, preferably predetermined, threshold. Furthermore, the method involves determining the user's intent to access an access point based on the changes noticed in the blood characteristics.

## Description

### TECHNICAL FIELD

The present invention relates generally to the field of computer-implemented methods for access control. More specifically, it relates to performing access control using a wearable device.

### BACKGROUND ART

The field of access control is rapidly evolving with the advancement in technology. Key cards, passwords, and other traditional methods of access control are becoming obsolete due to their vulnerability to breaches and their insufficiency in addressing the changing needs of individual and organizational security. Physical biometrics like fingerprints, facial recognition, and more recently, vein patterns, have come up as a viable solution to these concerns. However, these methods often rely on a single data point of comparison, rendering them susceptible to security breaches.

In parallel, the use of wearable devices, such as smartwatches and smart rings, infiltrated into every aspect of life, including security protocols. These wearables often include sensors that track a variety of biometric data, providing a rich source of personal information. However, most wearables are mainly used for tracking physical health and fitness and have not been substantially incorporated into access control systems.

The systems that use wearables as part of their verification protocol usually require the user to perform an action to verify identity, such as pressing a button to send a signal. This could prove inconvenient to the user, particularly if they are carrying items or their hands are otherwise preoccupied. Moreover, implementing an authentication signal that requires user interaction can increase the chance of accidental or unauthorized activations.

The conventional methods of linking changes in physiological attributes to intent or specific actions are complex and may involve significant processing power, often requiring bespoke algorithms and a substantial amount of time-bound, experiential training data. Such complexity can hinder the proliferation of sophisticated biologically-driven access control systems.

Currently used methods of wireless data transmission for access control, like Bluetooth or NFC, have their own limitations. They can be susceptible to cyber-attacks when not implemented properly and might not work from certain distances or without a clear line of sight. Moreover, these systems usually involve sending a steady signal, which can be intercepted or copied.

Therefore, there is an urgent need for an advanced access control method and system that would utilize the potential of wearable technology effectively to provide more integrated, intuitive, and secure access control solutions.

### SUMMARY OF INVENTION

The problem is solved by the subject-matter defined in the independent claims. Advantageous modifications of embodiments of the invention are defined in the dependent claims as well as in the description and the figures.

According to a first aspect of the present invention, a computer-implemented access control method is provided. The method may be performed by a wearable device. The wearable device may be attached to a user's body part, in particular a user's arm and/or a user's hand. The method may comprise continuously or repeatedly obtaining sensor data of a biological sensor of the wearable device, wherein the sensor data includes biological characteristics of the user. The method may comprise determining a change in the biological characteristics, wherein the magnitude of the change exceeds at least one threshold. The method may comprise determining a user's intent to access a access point based on the determined change in the biological characteristics.

The access point is preferably a physical access point, e.g., a door, a gate and/or the like.

This approach uses a wearable device incorporating biological sensors to recognize a user's intent to gain access, providing for a more intuitive, sophisticated, and secure method of access control. In the forthcoming sections, more preferred embodiments and benefits of this approach will be elaborated upon in greater detail.

The expression "physical access point" may be understood covering all kinds of physically accessible points, such as a room in a building, a secured area and/or the like. The physical access point may in particular include a door, a gate, a turnstile, a revolving door, a sliding door, an entrance element equipped with information indicators indicating if access is permitted or not, and/or the like.

The expression "wearable device" can be understood as a category of technology gadgets designed to be worn or attached to the user's body. These devices, encompassing a wide range of capabilities and uses, are typically composed of smart sensors and utilize connectivity, generally through Bluetooth or Wi-Fi, to exchange data with a user's smartphone or computer. Examples of wearable devices may include smartwatches, fitness trackers, smart glasses, wearable cameras, smart clothing, medical devices, and even smart jewelry. Slightly more specific, in the context of this invention, a wearable device is one which can read, obtain and analyze biological data from the wearer, and participate in the access control method, e.g., by sending access credentials and other related information.

The expression "user's body part" may be understood in general terms as any part or portion of a user's body. This may refer to something as broad as an entire limb or as specific as a fingertip. In this invention's context, the emphasis is made on the arm, hand, or fingers where the wearable device is usually worn or attached. This allows the device to readily and continuously monitor biological parameters without impeding the user's regular activities.

The expression "sensor data" can be comprehended as the raw, typically numerical, information that a sensor in a device collects. In the context of the present invention, sensor data refers to the biological information that the wearable device's sensor collects from the user's body part, such as data associated with the user's blood characteristics, with the user's electrical skin impedance and/or the like.

The expression "biological sensor" can be understood broadly as any type of sensor that can measure a biological function or marker. Within the context of this invention, the biological sensor refers to a sensor housed within the wearable device, capable of capturing information about the biological charateristics of the user, e.g. the user's blood characteristics.

The phrase "in a continuous or repetitive manner" can be understood as an ongoing or repeated process over time. This is generally meant to indicate that data is collected at regular intervals as opposed to one-off or sporadic data capture. The term, applied in this context, suggests that the wearable's biological sensor is consistently or recurrently recording sensor data from the user. This continuous stream of data allows for more effective monitoring and accurate determination of changes in the user's blood characteristics.

The expression "biological characteristics" may include measurable or identifiable attributes or properties of the user. More specific, these characteristics could relate to data that a wearable device with a biological sensor could realistically capture, like blood flow patterns, speed, pressure and possibly oxygenation levels.

The expression "change in the biological characteristics" can be understood as any meaningful or consistent variation, fluctuation, or alteration in the user's biological characteristics, e.g., blood characteristics, as captured by the biological sensor. In the context of this invention, a change in these characteristics could be something induced by the user's intent to access an access point, for example, a change in blood flow or pressure triggered by a specific gesture.

The expression "magnitude of the change" refers to the size, extent, or amount of change in the characteristics of the user's blood. This terminology, within the scope of this invention, is particularly about the extent of this change being significant enough to pass a threshold and thus be counted as indicating a potential intent to access.

The term "threshold" can be understood as a preset level or limit that when reached or exceeded, triggers a specific action or response. Within this invention, the threshold relates to a pre-determined level of change in the user's blood characteristics. If the magnitude of the change exceeds this threshold, it would consequently point to the user's intent to access. Exceeding may include exceeding below or above the threshold.

The expression "user's intent to access" may be understood as the user's will or purpose to gain entry or access some controlled entity, i.e., an access point.

The concept according to the present invention provides a new perspective in the implementation and advancement of secured systems, rendering it an efficient and reliable solution compared to existing alternatives. In particular, the security level is higher due to the use of biometrical data, and the access control can be performed more convenient, i.e., almost seamlessly through a simple gesture. A specific gesture will for example result in a specific change of the user's blood characteristics which is unique to the user and thus nobody can copy this. A gesture is more convenient than taking you a mobile phone and/or interacting with a reader at the wall next to an access point.

The proposed method uses sensor data from the wearables and a corresponding biological interpretation of relevance to the user's intent for access. It is a novel implementation that underscores the future of cybersecuring physical and digital assets.

Tagged as one of the many advantages of this invention is the commendable combination of technology and biometrics to usher in an era of secure access control. Traditional access control methods have several potential pitfalls, such as potential loss, theft, or cloning of access cards, forgotten or easily guessable passwords, and the inconvenience of carrying around physical keys. This invention neatly bypasses these issues through the clever utilization of wearable technology. By employing wearable devices that are commonly used in everyday life, such as smartwatches or fitness bands, it minimizes the burden of carrying around separate devices or remembering complex codes for access control, which heightens user convenience considerably.

The technique of determining access based on changes in biological characteristics derived through the wearable device's sensor occupies a prime position. This unique approach, favoring the user's biological information, presents a unique ID that cannot be lost, stolen, or easily duplicated. The characteristics used could potentially be unique to each user and are extremely difficult to forge, thus providing an access control method with a heightened level of security.

Furthermore, the method of continuous or repetitive monitoring of the user's biological characteristics underscores an added advantage of real-time detection. The system does not rely on a one-off measurement but continues to check and analyze over time, providing a more comprehensive view of the user's biometric responses. This continuous monitoring allows the system to track changes, observe trends, and possibly predict a user's intent to access even before the action is initiated.

Significantly, the use of a threshold to filter simple fluctuations from actual intent inducing changes adds to the invention's reliability. The system undertakes the access process only when the change exceeds a certain magnitude, ideally predetermined, thereby successfully avoiding false triggers or invasive actions based on irrelevant fluctuations.

The aspect of determining the user's intent to access based on biological characteristics infers a much more intuitive and user-friendly system. It offsets the need for the user to remember passwords or carry extra devices, enabling a more seamless interaction between the user and the secured system. The user may not even need to make a conscious decision to activate their access credential, enhancing the method's ease of use.

Moreover, the efficacy of the proposed method is unquestionable in terms of adaptability and scalability. The method can easily be incorporated into existing systems, both digital and physical, with only the integration of the biological sensor data interpretation as the additional requisite. Given the rapidly evolving ecosystem of wearable devices, the method can be adopted widely with little need for major infrastructural changes affecting both users and the access control system providers.

Lastly, the emphasis on machine engagement, corresponding to the extraction and use of biological data, broadens the platform for contactless methods, a significant element in light of ongoing health and hygiene concerns.

In summation, this computer-implemented access control method constitutes a progressive stride in the real of secure access control systems. Its blend of biological data, wearable technology and intuitive access intends defiantly carves an evolutionary roadmap for the access control verticals. The method extends beyond the conventional norms, affirming a technology that is not just secure, but user-centric and adaptive to the changing spectrum of global needs.

It may be provided that the computer-implemented method further includes sending, or even broadcasting, an access credential as a consequent to determining the user's intent to access. The sending and/or broadcasting may be performed by the wearable, or alternatively be triggered by the wearable, e.g., by triggering a connected portable device of the user to send the access credential.

These features present a notable advantage in the field of access control: the addition of broadcasting or sending access credentials as a result of determining the user's intent to access. This feature constitutes a powerful, user-friendly, and efficient method for streamlining the access process. Firstly, the ability to send or broadcast access credentials directly from the wearable device expedites the access control process. It simplifies the user's need to remember complex codes, keys, or carry additional devices or cards for gaining access. The broadcasted signals could potentially cover a larger area compared to traditional access control systems that often require close contact or direct line of sight. Thus, this feature reduces the necessity for the user to be physically close to the access point, improving convenience and ease of use. Secondly, the fact that this credential broadcasting occurs when the user's intent to access is determined exhibits a dynamic, intuitive system. The control system is not merely static, waiting for a pass-code or swipe card. Instead, it's actively involved in reading and interpreting user intent, then reacting appropriately. This proactive approach could potentially improve response times and system interactions, providing a smoother user experience.

Furthermore, the provision for the alternative triggering of a connected portable device by the wearable to send the access credential presents another layer of flexibility and adaptability. This grants the users the ability to choose their preferred device for access control based on their convenience, hence enhancing usability. For example, if the battery of the wearable is low, the user can switch to a portable device like their smartphone to ensure uninterrupted access control.

In essence, this promotes a seamless, intuitive, and highly adaptable access control method that enhances security while significantly improving the user experience. By integrating the biological data interpretation with user-friendly technology, this invention presents an access control system that is well-suited for the digital age.

It may be provided that the computer-implemented method further involves transmitting, particularly broadcasting, additional authorization information upon establishing the user's intent to access.

This introduces a layer of complexity and security not available in traditional access control systems, thereby enhancing the technical effects and advantages. The additional authorization information can be used as a supplementary measure to not only verify the user's identity but also to furnish context-specific or time-sensitive information that can be used to enhance the security and user conformity of the access system. This innovation in access control provides an array of technical effects, broadening the resilience and adaptability of security systems. The ability to broadcast additional authorization information ensures elaborative communication, especially in environments involving multi-factor authentication. This capability can enrich the access control system by providing additional or time-sensitive context information, adding prevailing conditional layers to the authorization process.

Broadcasting over a network spreads the data across multiple nodes, ensuring that any device within the network that is authorized to listen and react to such data can do so, making the system robust against specific device failure. For instance, if one device responsible for holding or relaying the access control information fails, the bredth of the broadcast allows alternative devices within the network to pick up the slack.

It may also facilitate user movement through multiple access points within a larger system. The additional information can act as a digital trail, vital throughout the expanse of a multipoint access system, preventing repetitive authorization requests. The user gets the benefit of a smoother, 'single-sign-on' type experience, even in a physical, multi-access-point environment.

The advantages provided by this additional authorization information flow extend beyond security. They also improve the user experience as the system can dynamically react to the user's intent to access, rather than being tied purely to a reactive model of waiting for a user command. The system can 'anticipate' user needs and adapt to meet them.

In conclusion, the transmission or broadcasting of additional authorization information imposes an advanced structure to an otherwise one-dimensional system. It not only fortifies the access control landscape but imparts a user-friendly atmosphere, an accomplished feat in synchronizing effortless access in tow with stringent security.

It may be provided that within the computer-implemented method the additional authorization information comprises at least one of the following: a clock time, a time period where access is permitted, a specific point in time where access is granted, a device ID, or user-related information.

This altogether allows for a nuanced, context-aware access control system that surpasses the capabilities of many existing solutions. Starting with clock time as part of the access control' parameters raises understandability for a more dynamic, real-time bound access control protocol. By adjusting authorization according to the real time, this feature introduces another layer to security management. For example, a system can be programmed to only allow access during specific hours of the day. This can be particularly useful for corporate settings where access to certain areas is restricted outside of working hours. Similarly, by incorporating a specified time period where access is granted, the system can manage access in a context that stretches beyond the immediate moment. This might be particularly beneficial for temporary access, such as visits by guests, contract workers, or for employees with time-bound clearance to access certain secure areas. It might also help in maintaining compliance with legal restrictions, such as labor law rules about the maximum length of work shifts. The ability to pinpoint a specific time when access is permitted provides a powerful tool for exact scheduling. This could be used to enhance security during particularly sensitive operations, or to help manage and stagger user access for high-demand resources.

Including device ID as a part of the additional authorization information heightens the security by tying the access not just to the user's biometrics but also to a specific device. It's an added layer of protection against possible security breaches. In this regard, even if the biometric information was somehow compromised, without the corresponding device ID, access would be denied.

Finally, the allocation of authorization information to the user's side makes it possible to install simple access control readers which are not carrying large amounts of information regarding that authorization information, e.g., as a list of time periods allocated to device IDs and/or the like. Thus, the system becomes very flexible and easy to maintain.

It may be provided that in the computer-implemented method, the biological characteristics of the user includes information about blood characteristics of the user's blood circulating in the body part, in particular data on blood pressure information and/or blood flow associated with the user.

The ability to measure particular biological attributes provides a distinctive advantage to the system, enhancing both its functionality and versatility. For example, a spike in blood pressure might indicate an intent to access, prompting the system to prepare for authentication. The spike may be a result of a gesture being performed. In addition, using blood characteristics such as blood pressure and blood flow contributes an underscored level of security to the access control system. As these features are unique and internal to each individual, they are extremely difficult to replicate, cloning, or forge. In that regard, this implementation makes for a more secure system compared to those that base access on external, easily mimicked attributes. Furthermore, these measurements can be gathered non-invasively through wearable technology already on the market. This use of existing, widely adopted technology can lead to increased user acceptance of the system and more comfortable, habitual use.

It may be provided that a change in the biological characteristics, in particular of the user's blood, is determined when the user performs a predetermined gesture, the gesture causing the change. Further, it may be provided that the gesture is performed by the user's body part where the wearable device is attached, in particular with the user's hand and/or arm and/or one or more fingers.

With respect to the particular embodiment of determining a change in the characteristics of the user's blood when they perform a predetermined gesture with the body part where the wearable device is attached, particular benefits arise. This approach puts forth a distinctive blend of biometric data and gestural cues, which has clear technical advantages and offers an innovative yet practical approach to secure access control. Initiating the gesture recognition protocol enhances system interaction by providing a unique, personalized user experience. It allows the user to instigate seamless actions, such as opening a door or logging into a system, through natural movements. There's no need for the user to pause and swipe a card or type in a password, as the system anticipates intent via the gesture. This kind of user-friendly, intuitive interface can increase the acceptance and adoption of the system by a range of user groups. By tying the recognition of a gesture to a change in the user's blood characteristics, the system can enhance the identification process's effectiveness and security. Gestures can often be mimicked, but when coupled with distinctive biological markers apparent through blood characteristics changes, they become incredibly challenging to replicate. This, in turn, reduces the risk of unauthorized access by malicious parties.

The concept of using gestures as access triggers also opens up the potential to customize and personalize the access control system further. Since users can have unique gestures tailored to their comfort and preferences as access triggers, the users are offered a high level of customization, fostering a sense of ownership and increased user satisfaction.

It may be provided that different gestures and corresponding changes in the biological characteristics of the user, in particular of the user's blood, are allocated to different access points, thus enabling the user to select an access point that is intended to be accessed by selecting a specific gesture.

An additional benefit lies in the use of a variety of gestures that could correspond to different actions or access levels. For example, a particular gesture could grant complete access, another could provide limited access, and yet another could signal a distress call. Such fine-tuned responsiveness creates a more versatile and functionally rich access control environment.

In other words: It is particularly preferred that multiple gestures are predefined, each of which indicates a distinguishable access-point-specific intention of the user to access a respective access point.

This has several advantages particularly with its capability to predefine multiple gestures, each indicating a distinguishable "access-point-specific" intention of the user to access a respective access point.

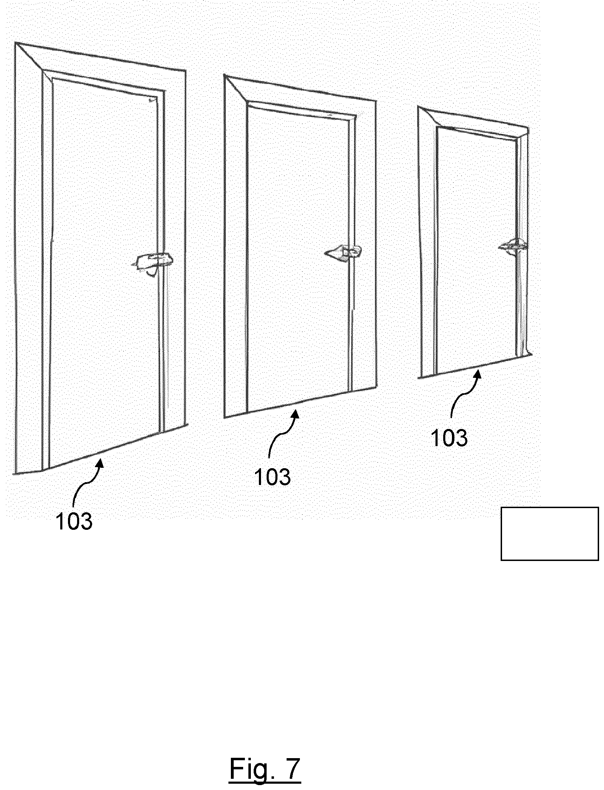

First, it enhances the security with an adoption of an individualistic approach to authenticate a user based on distinctive blood characteristics, observed from the sensor data. Further, it provides the possibility to open one specific access point 103 out of a plurality of access points 103, i.e., in case of three access points 103 as illustrated in figure 7, it each access point 103 may have an allocated specific gesture in order to open only the one intended access point 103. Further, it provides a more personalized experience as the multiple gestures may be customizable. Thus, users can define unique gestures to represent their intention for different access points 103, offering a highly personalized experience.

It may be provided that, prior to performing the step of continuously or repeatedly obtaining sensor data, it is performed a step of receiving training user input by the wearable device, wherein the wearable device is set in a training mode enabling the user to train particular gestures to the wearable device and/or to define particular gestures and/or allocate particular gestures to specific access point(s).

It may be provided that in the computer-implemented method, the process of determining the change in the biological characteristics, in particular of the user's blood, is executed by a machine learning model, optionally with the machine learning model having previously been trained by the user.

This advanced incorporation of artificial intelligence within the access control procedure brings about several noteworthy technical effects and advantages. Firstly, the use of machine learning facilitates the continuous improvement and optimization of the access control system automatically. As machine learning algorithms rely on patterns and inference, the system can learn from each interaction, better understanding the correlation between blood characteristics changes and user intent over time. This ability to evolve and adapt, even without direct human intervention, makes for a highly efficient system that becomes more accurate and reliable with each use.

Due to the vast complexities surrounding biological data, having a machine learning model process and interpret these signals is hugely beneficial. It simplifies the task of identifying underlying patterns or correlations that might not be immediately apparent. For instance, slight changes in blood pressure or pulse patterns in response to specific gestures might be hard to spot using traditional programming but could be easily recognized by a machine learning model.

In terms of user experience, the potential option for users to train the machine learning model adds a personalized dimension to the system. By learning from the data specific to each user, the model can tailor its responses to the individual. This allows for a highly personalized response to each user's specific physiological responses and gestures, further increasing the system's efficacy and user-conformity.

It may be provided that in the computer-implemented method, the act of dispatching the access credential, and preferably sending the further authentication information is carried out with a wireless transmitter of the wearable device, preferably employing technologies like Bluetooth, Bluetooth low energy and/or ultra-wideband.

It may be provided that the computer-implemented method further involves performing ultra-wideband transmissions with peripheral devices to gain positioning and/or movement information of the user, in which case sending the access credential and/or further authentication information is performed only upon establishing a further user's intent to access the access point based on the obtained positioning and/or movement detail of the user.

Performing ultra-wideband transmissions with peripheral devices to obtain positioning and/or movement information of the user is commonly known as "ranging". Sending the access credential and/or further authentication information is performed only upon establishing a further user's intent to access the access point based on obtained positioning and/or movement details. This element of using positioning and movement data in addition to the above-described intent detection based on the biological sensor data can be termed as "double intent detection". This function garners two independent sets of data - one from the user's blood characteristics and another from the user's positioning and/or movements. The marvel of double intent detection lies in its ability to amass and collate these two types of data to provide a more substantial and coherent determination of the user's access intent. The first layer of intent detection analyses the changes in blood characteristics. Once the predetermined threshold is crossed, it signals an intent to access. Simultaneously, the system harnesses the ultra-wideband transmissions to collect positioning and/or movement data of the user, forming the second layer of intent detection.

Positioning data could entail whether the user is in proximity to the access point and the relative distance or direction of movement towards it. Recognizing such positioning or movement patterns can suggest the intention to interact with the access point. Only upon establishing this additional intent to access, based on ultrawide band gathered information, does the system send the necessary access credential or further authentication information.

This double intent detection provides a fail-safe measure, enhancing the security and reliability of the system. Instead of relying solely on a single data point - like solely on blood characteristics or just the positioning of the user - the method uses an intersection of both data sets to guarantee that the inferred intent to access is accurate and reliable.

Moreover, using ultra-wideband transmissions implies a greater ability to work securely in diverse environments and situations. These transmissions provide highly accurate, real-time positioning and movement data, making the system adaptable in varied settings. Beneficially, the access credential may be sent via ultra-wideband either.

It may be provided that the method further comprises tracking and evaluating the user's movement, in particular a stoppage of the user where the user essential stands still. The determining the user's intent to access the physical access point may be performed further based on the movement information. Optionally, the user's intent to access the physical access point is only determined if a stoppage of the user is detected in addition to determining the change in the biological characteristics.

By this, the precision of detecting the intent is further enhanced. In particular, a user essentially stopping a walking movement may be a further intent indication that may be advantageously used.

According to a second aspect of the present invention, an electronic device, in particular a wearable device, preferably a smartwatch and/or a smart ring, being configured to perform a method according to the first aspect of the present invention, is provided.

It may be provided that the electronic device includes: a biological sensor being capable of obtaining sensor data relating to characteristics of a user's blood; at least one processor being configured to process obtained sensor data and determine changes in the characteristics of the user's blood; at least one memory storage being configured to store access credentials and/or further authorization information, wherein the further authorization information preferably includes one or more of the following: a clock time, a time period where access is permitted, a point in time where access is permitted, a device ID, user-related information; and at least one transmitter being configured to wirelessly send, preferably broadcast, the access credential and/or the further authorization information.

It may be provided that the at least one transmitter includes an ultra-wideband transceiver being configured to perform ultra-wideband transmission with peripheral devices, such as ultra-wideband anchors.

According to a third aspect of the present invention, an access control system is provided, including an electronic device according to the second aspect of the present invention and including an access controller being configured to operate an access point, in particular to grant and/or deny access to a user based on received access credentials and/or further authentication information.

According to a fourth aspect of the present invention, a use of a machine learning model in a method according to the first aspect of the present invention, is provided.

According to a fifth aspect of the present invention, a computer program comprising computer program code is provided, which, when run on an electronic device according to the second aspect of the present invention, causes the electronic device to perform a method according to the first aspect of the present invention.

All features, technical implementation details and advantages described with respect to any one of the aspects of the present invention described herein are self-evidently mutatis mutandis applicable for any one of the other aspects of the present invention and vice versa.

### BRIEF DESCRIPTION OF THE DRAWINGS

The disclosure may be better understood by reference to the following drawings:
Fig. 1: A first schematic illustration of a user's hand performing a gesture according to embodiments of the present invention.
Fig. 2: A second schematic illustration of a user's hand performing a gesture according to embodiments of the present invention.
Fig. 3: A third schematic illustration of a user's hand performing a gesture according to embodiments of the present invention.
Fig. 4: A fourth schematic illustration of a user's hand performing a gesture according to embodiments of the present invention.
Fig. 5: A fifth schematic illustration of a user's hand performing a gesture according to embodiments of the present invention.
Fig. 6: A sixth schematic illustration of a user's hand performing a gesture according to embodiments of the present invention.
Fig. 7: A schematic illustration of access points according to embodiments of the present invention.
Fig. 8: A flow chart diagram including steps according to a method of embodiments of the present invention.

### DETAILED DESCRIPTION OF THE DRAWINGS

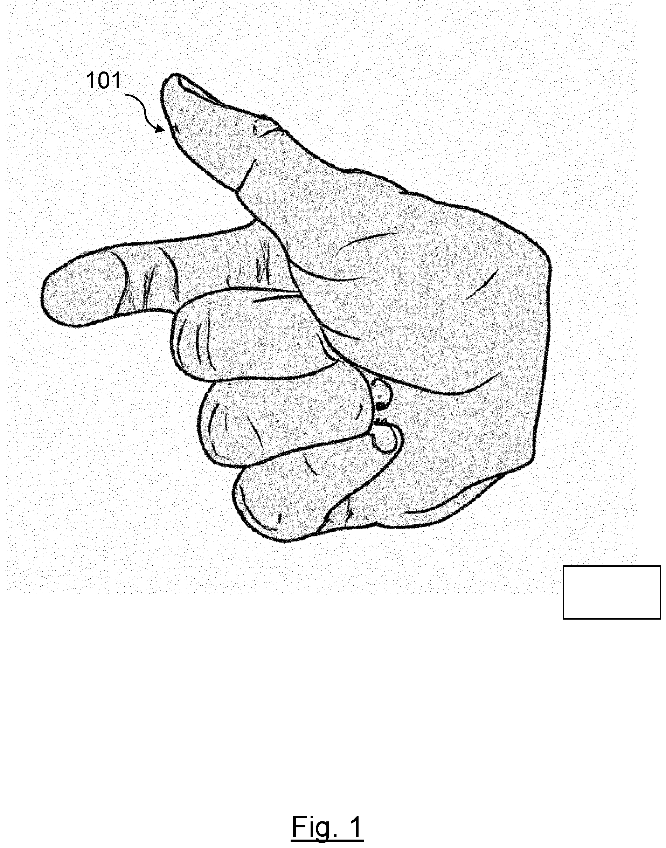

Figures 1, 2, 3, 4, 5 and 6 each depict a user's body part 101 wearing a wearable device 102 being a smartwatch. The user's body part 101 is, in each case, performing a gesture which causes a change in the characteristics of the user's blood. The wearable device 102 performs continuously or repeatedly obtaining sensor data of a biological sensor of the wearable device 102, wherein the sensor data includes information about blood characteristics of the user's blood circulating in the body part 101 and/or wherein information about blood characteristics of the user's blood circulating in the body part 101 is determined based on the sensor data. A user's intent to access an access point 103 is determined based on the determined change in the characteristics of the user's blood.

A change in the characteristics of the user's blood is determined when the user performs a predetermined gesture, as describes above, with his/her body part 101 where the wearable device 102 is attached, in particular with his/her hand and/or arm and/or one or more fingers, wherein the gesture preferably causes the change.

Determining the change in the characteristics of the user's blood may be performed by a machine learning model run on the wearable device 102, optionally the machine learning model being trained by the user in beforehand.

In a preferred embodiment, it may be provided that the change in the characteristics of the user's blood and/or the characteristics of the user's blood in general, independent from any change, is unique to the user and thus allows distinct and unmistakable identification of the user. Thus, apart from detecting the intent of the user, this unique information may be used to identify the user and perform an access control decision, i.e., granting the user access or not, dependent on the identity of the user. This significantly raises the security of the system.

In Figure 1, the index finger and thumb are tapped on each other as part of the gesture, in particular several times, preferably 2 times. Such a gesture can be referred to as a "double tap" gesture. It is also conceivable to tap three times, which can be referred to as a "triple tap".

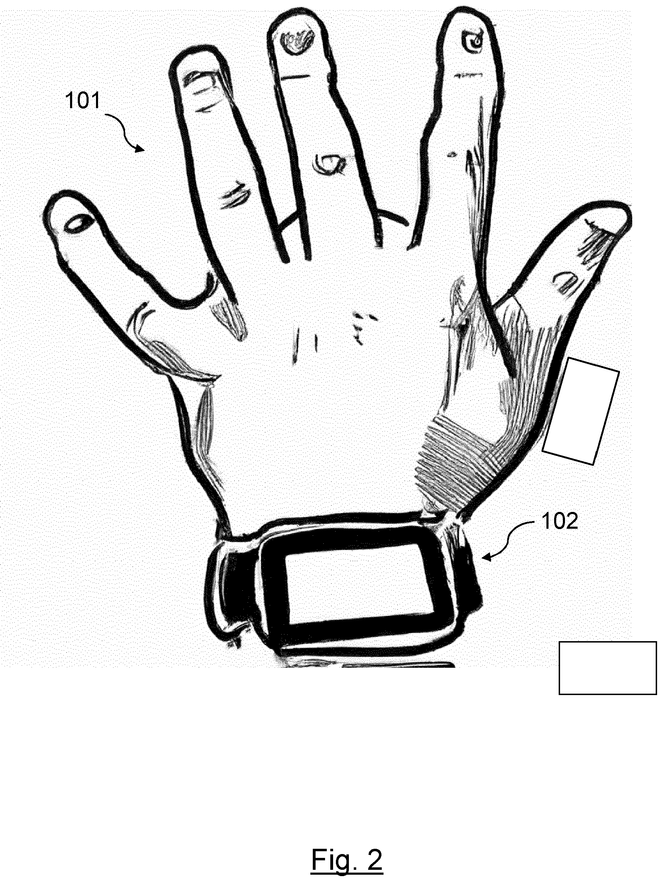

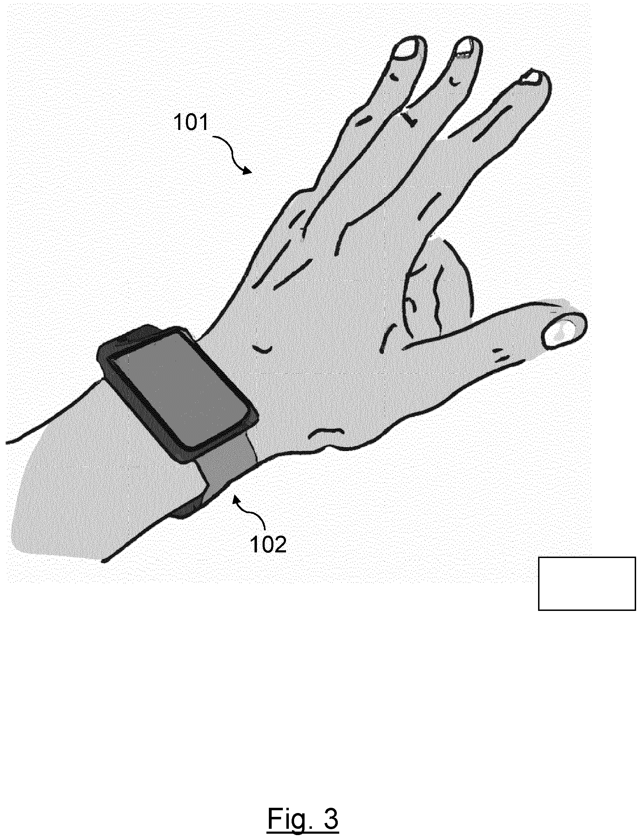

In Figure 2, all fingers of the hand are spread as part of the gesture. In Figure 3, certain fingers are spread.

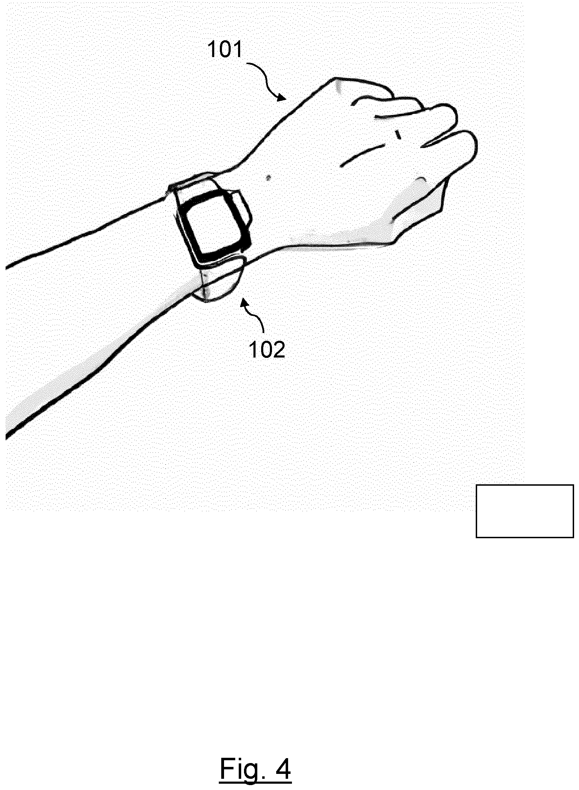

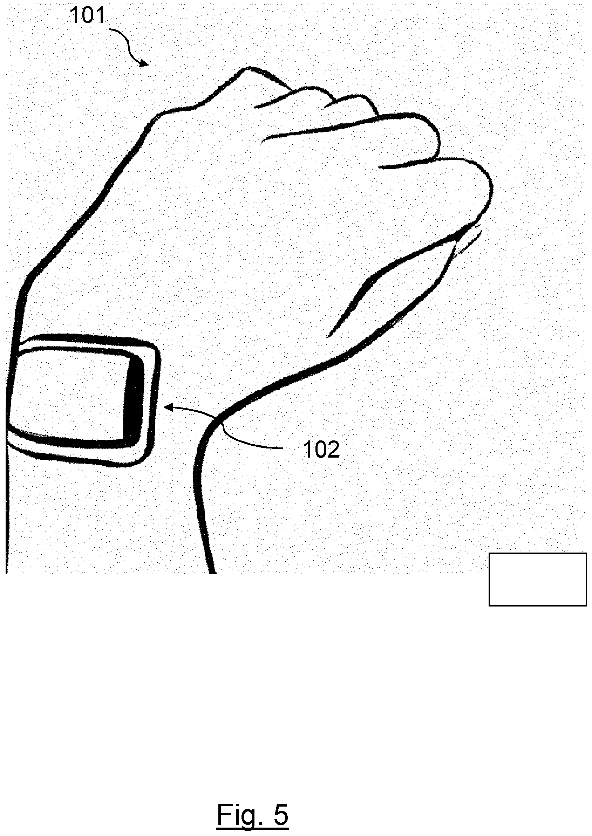

In Figure 4, a fist is formed, i.e., the fingers are pressed together. In Figure 5, in addition to forming the fist, the wrist is bent.

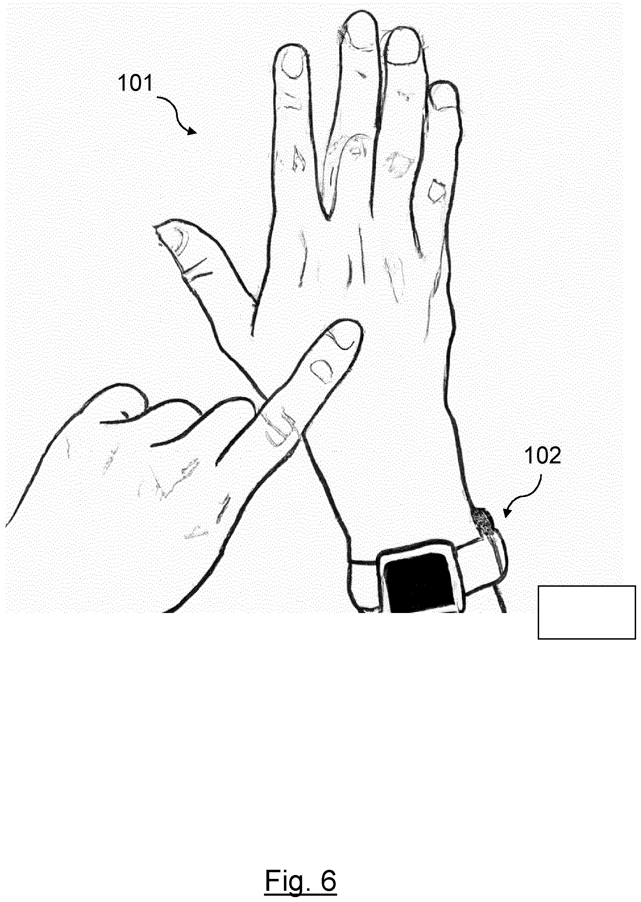

In Figure 6, one hand is tensed and the other hand is tapped on the back of the hand.

All these gestures have an influence on the characteristics of the user's blood measured at runtime, in particular the blood pressure.

It is particularly preferred that multiple gestures are predefined, each of which indicates a distinguishable access-point-specific intention of the user to access a respective access point.

This has several advantages particularly with its capability to predefine multiple gestures, each indicating a distinguishable "access-point-specific" intention of the user to access a respective access point.

First, it enhances the security with an adoption of an individualistic approach to authenticate a user based on distinctive blood characteristics, observed from the sensor data. Further, it provides the possibility to open one specific access point 103 out of a plurality of access points 103, i.e., in case of three access points 103 as illustrated in figure 7, it each access point 103 may have an allocated specific gesture in order to open only the one intended access point 103. Further, it provides a more personalized experience as the multiple gestures may be customizable. Thus, users can define unique gestures to represent their intention for different access points 103, offering a highly personalized experience.

Figure 7 shows three access points 103 in the form of doors. Each of the doors has an access controller that can receive and/or send commands and/or data from the wearable device 102 and/or from a portable user device connected to the wearable device 102. Such commands and/or data may include, for example, access credentials, information about the intent of the user, or the like.

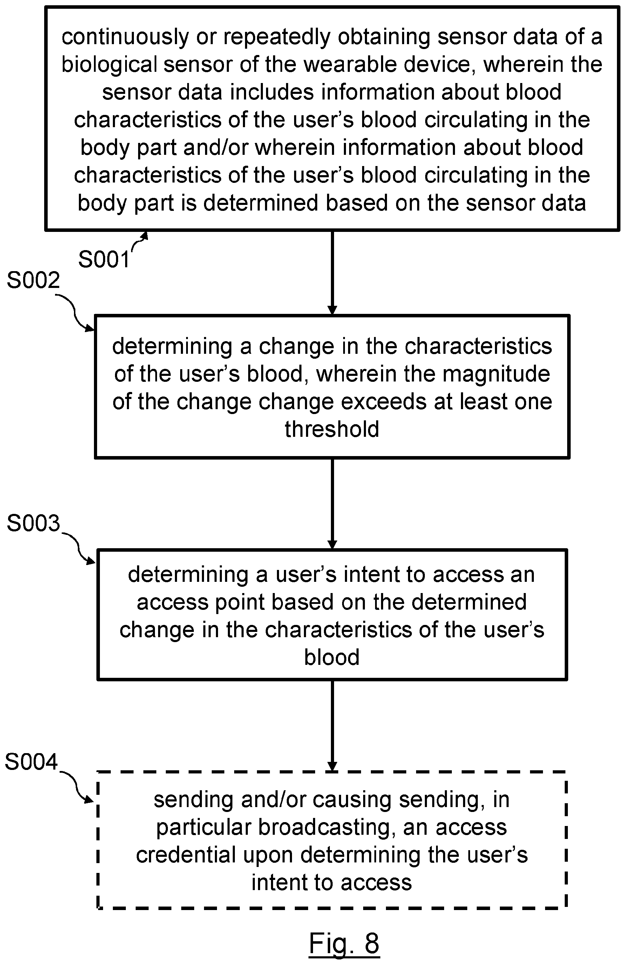

Figure 8 includes steps of the present invention, in particular steps S101, S102, S103 and S104. Step S104 is an optional step and is thus illustrated with broken lines.

### Machine Learning

Certain embodiments of the invention may be based on using a machine learning model or machine learning algorithm. Machine learning may refer to algorithms and statistical models that computer systems may use to perform a specific task without using explicit instructions, instead relying on models and inference. For example, in machine learning, instead of a rule-based transformation of data, a transformation of data may be used that is inferred from an analysis of historical and/or training data. For example, the content of images may be analyzed using a machine learning model or using a machine learning algorithm. In order for the machine learning model to analyze the content of an image, the machine learning model may be trained using training images as input and training content information as output. By training the machine learning model with a large number of training images and/or training sequences (e.g. words or sentences) and associated training content information (e.g. labels or annotations), the machine learning model "learns" to recognize the content of the images, so the content of images that are not included in the training data can be recognized using the machine learning model. The same principle may be used for other kinds of sensor data as well: By training a machine learning model using training sensor data and a desired output, the machine learning model "learns" a transformation between the sensor data and the output, which can be used to provide an output based on non-training sensor data provided to the machine learning model. The provided data (e.g., sensor data, meta data and/or image data) may be preprocessed to obtain a feature vector, which is used as input to the machine learning model.

Machine learning models may be trained using training input data. The examples specified above use a training method called "supervised learning". In supervised learning, the machine learning model is trained using a plurality of training samples, wherein each sample may comprise a plurality of input data values, and a plurality of desired output values, i.e., each training sample is associated with a desired output value. By specifying both training samples and desired output values, the machine learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g., a classification algorithm, a regression algorithm or a similarity learning algorithm). Classification algorithms may be used when the outputs are restricted to a limited set of values (categorical variables), i.e., the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters. Reinforcement learning is a third group of machine learning algorithms that may be used to train the machine learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine learning algorithms. For example, feature learning may be used. In other words, the machine learning model may at least partially be trained using feature learning, and/or the machine learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e., outlier detection) may be used, which is aimed at providing an identification of input values that raise suspicions by differing significantly from the majority of input or training data. In other words, the machine learning model may at least partially be trained using anomaly detection, and/or the machine learning algorithm may comprise an anomaly detection component.

In some examples, the machine learning algorithm may use a decision tree as a predictive model. In other words, the machine learning model may be based on a decision tree. In a decision tree, observations about an item (e.g., a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine learning algorithms. In other words, the machine learning model may be based on one or more association rules. Association rules are created by identifying relationships between variables in large amounts of data. The machine learning algorithm may identify and/or utilize one or more relational rules that represent the knowledge that is derived from the data. The rules may e.g. be used to store, manipulate or apply the knowledge.

Machine learning algorithms are usually based on a machine learning model. In other words, the term "machine learning algorithm" may denote a set of instructions that may be used to create, train or use a machine learning model. The term "machine learning model" may denote a data structure and/or set of rules that represents the learned knowledge (e.g., based on the training performed by the machine learning algorithm). In embodiments, the usage of a machine learning algorithm may imply the usage of an underlying machine learning model (or of a plurality of underlying machine learning models). The usage of a machine learning model may imply that the machine learning model and/or the data structure/set of rules that is the machine learning model is trained by a machine learning algorithm.

For example, the machine learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g., of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine learning model may be a support vector machine, a random forest model or a gradient boosting model. Support vector machines (i.e., support vector networks) are supervised learning models with associated learning algorithms that may be used to analyze data (e.g., in classification or regression analysis). Support vector machines may be trained by providing an input with a plurality of training input values that belong to one of two categories. The support vector machine may be trained to assign a new input value to one of the two categories. Alternatively, the machine learning model may be a Bayesian network, which is a probabilistic directed acyclic graphical model. A Bayesian network may represent a set of random variables and their conditional dependencies using a directed acyclic graph. Alternatively, the machine learning model may be based on a genetic algorithm, which is a search algorithm and heuristic technique that mimics the process of natural selection.

### General aspects

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some or all of the method steps may be executed by (or using) a hardware apparatus, such as a processor, a microprocessor, a programmable computer or an electronic circuit. Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments of the invention provide a data carrier having electronically readable control signals, which are capable of cooperating with a programmable computer system, such that one of the methods described herein is performed.

Generally, embodiments of the invention can be implemented as a computer program (product) with a program code, the program code being operative for performing one of the methods when the computer program product runs on a computer. The program code may, for example, be stored on a machine-readable carrier. Other embodiments comprise the computer program for performing one of the methods described herein, stored on a machine-readable carrier. In other words, an embodiment of the present invention is, therefore, a computer program having a program code for performing one of the methods described herein, when the computer program runs on a computer.

A further embodiment of the invention provides a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention provides a data stream or a sequence of signals representing the computer program for performing one of the methods described herein. The data stream or the sequence of signals may, for example, be configured to be transferred via a data communication connection, for example, via the internet.

A further embodiment of the invention provides a processing means, for example, a computer or a programmable logic device, configured to, or adapted to, perform one of the methods described herein.

A further embodiment of the invention provides a computer having installed thereon the computer program for performing one of the methods described herein.

A further embodiment of the invention provides an apparatus or a system configured to transfer (e.g., electronically or optically) a computer program for performing one of the methods described herein to a receiver. The receiver may, for example, be a computer, a mobile device, a memory device, or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

## Claims

1. A computer-implemented access control method, the method being performed by a wearable device, the wearable device being attached to a user's body part, in particular a user's arm and/or a user's hand, the method comprising the steps of:
continuously or repeatedly obtaining sensor data of a biological sensor of the wearable device, wherein the sensor data includes biological characteristics of the user;
determining a change in the biological characteristics, wherein the magnitude of the change exceeds at least one threshold;
determining a user's intent to access a physical access point based on the determined change in the biological characteristics.

2. The computer-implemented method of claim 1, the method further comprising the step of:
sending and/or causing sending, in particular broadcasting, an access credential upon determining the user's intent to access.

3. The computer-implemented method of claim 2, the method further comprising the step of:
sending, in particular broadcasting, a further authorization information upon determining the user's intent to access.

4. The computer-implemented method of claim 3, wherein the further authorization information includes one or more of: a clock time, a time period where access is permitted, a point in time where access is permitted, a device ID, user-related information.

5. The computer-implemented method of any one of the preceding claims, wherein the biological characteristics of the user includes information about blood characteristics of the user's blood circulating in the body part, in particular blood pressure information and/or blood flow information associated with the user.

6. The computer-implemented method of any one of the preceding claims, wherein a change in the biological characteristics, in particular of the user's blood, is determined when the user performs a predetermined gesture, the gesture causing the change,
optionally wherein the gesture is performed by the user's body part where the wearable device is attached, in particular with the user's hand and/or arm and/or one or more fingers.

7. The computer-implemented method of any one of the preceding claims, wherein different gestures and corresponding changes in the biological characteristics of the user, in particular characteristics of the user's blood, are allocated to different access points, thus enabling the user to select an access point that is intended to be accessed by selecting a specific gesture.

8. The computer-implemented method of any one of the preceding claims, wherein determining the change in the biological characteristics, in particular of the user's blood, is performed by a machine learning model, optionally the machine learning model being trained by the user in beforehand.

9. The computer-implemented method of any one of the preceding claims, wherein sending the access credential and preferably sending the further authentication information is performed using a wireless transmitter of the wearable device, preferably via bluetooth, bluetooth low energy and/or ultra-wideband.

10. The computer-implemented method of any one of the preceding claims, further comprising:
performing ultra-wideband transmissions with peripheral devices for obtaining positioning and/or movement information of the user, wherein sending the access credential and preferably sending the further authentication information is only performed, if a further user's intent to access the access point is determined based on the positioning and/or movement information of the user.

11. The computer-implemented method of any one of the preceding claims, further comprising:
tracking and evaluating the user's movement, in particular a stoppage of the user where the user essential stands still;
wherein the determining the user's intent to access the physical access point is performed further based on the movement information;
optionally wherein the user's intent to access the physical access point is only determined if a stoppage of the user is detected in addition to determining the change in the biological characteristics.

12. An electronic device, in particular a wearable device, preferably a smartwatch and/or a smart ring, being configured to perform a method according to one of claims 1-11.

13. The electronic device of claim 12, including:
a biological sensor being capable of obtaining sensor data relating to characteristics of a user's blood;
at least one processor being configured to process obtained sensor data and determine changes in the characteristics of the user's blood;
at least one memory storage being configured to store access credentials and/or further authorization information, wherein the further authorization information preferably includes one or more of the following: a clock time, a time period where access is permitted, a point in time where access is permitted, a device ID, user-related information;
at least one transmitter being configured to wirelessly send, preferably broadcast, the access credential and/or the further authorization information.

14. An access control system including an electronic device according to one of claims 12-13 and including an access controller being configured to operate an access point, in particular to grant and/or deny access to a user based on received access credentials and/or further authentication information.

15. A computer program comprising computer program code which, when run on an electronic device according to any one of claims 11-12, causes the electronic device to perform a method according to any one of claims 1 to 10.
